(19) Europäisches Patentamt / European Patent Office / Office européen des brevets

(11) **EP 2 741 758 B1**

(12) **EUROPEAN PATENT SPECIFICATION**

(45) Date of publication and mention of the grant of the patent:
**20.04.2016 Bulletin 2016/16**

(21) Application number: **12762087.0**

(22) Date of filing: **07.08.2012**

(51) Int Cl.:
***A61K 36/185*** *(2006.01)*

(86) International application number:
**PCT/PL2012/000063**

(87) International publication number:
**WO 2013/022357 (14.02.2013 Gazette 2013/07)**

(54) **POLYPHENOLIC FORMULATION, ITS PREPARATION AND USE**

POLYPHENOLFORMULIERUNG, IHRE HERSTELLUNG UND IHRE VERWENDUNG

FORMULATION POLYPHÉNOLIQUE, SA PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.08.2011 PL 39589711**
**08.08.2011 PL 39589811**
**08.08.2011 PL 39589911**

(43) Date of publication of application:
**18.06.2014 Bulletin 2014/25**

(73) Proprietors:
- **Uniwersytet Medyczny W Lodzi**
  **90-419 Lodz (PL)**
- **Politechnika Lódzka**
  **90-924 Lódz (PL)**
- **Uniwersytet Lodzki**
  **90-131 Lodz (PL)**

(72) Inventors:
- **ROZALSKI, Marcin**
  **PL-92-008 Lodz (PL)**
- **GOLANSKI, Jacek**
  **PL-90-145 Lodz (PL)**
- **WATALA, Cezary**
  **PL-95-200 Pabianice (PL)**
- **LABIENIEC, Magdalena**
  **PL-95-200 Pabianice (PL)**
- **KOZIOLKIEWICZ, Maria**
  **PL-93-355 Lodz (PL)**
- **PODSEDEK, Anna**
  **PL-94-041 Lodz (PL)**
- **SOSNOWSKA, Dorota**
  **PL-92-434 Lodz (PL)**
- **REDZYNIA, Malgorzata**
  **PL-93-335 Lodz (PL)**
- **PRZYGODZKI, Tomasz**
  **PL-93-380 Lodz (PL)**
- **LUZAK, Boguslawa**
  **PL-92-008 Lodz (PL)**
- **BONCLER, Magdalena**
  **PL-91-852 Lodz (PL)**

(74) Representative: **Kaminski, Piotr**
**Kaminski & Partners**
**Gerbera 14/13**
**05-500 Piaseczno (PL)**

(56) References cited:
**WO-A1-2005/058336**
**WO-A2-2009/023710**
**DE-A1- 19 939 350**
**US-A1- 2005 042 318**
**US-A1- 2009 291 071**

- **ANIOL MIROSLAW ET AL: "Extraction of Spent Hops Using Organic Solvents", JOURNAL OF THE AMERICAN SOCIETY OF BREWING CHEMISTS, AMERICAN SOCIETY OF BREWING CHEMISTS, ST PAUL, MN, US, vol. 66, no. 4, 1 January 2008 (2008-01-01), pages 208-214, XP009165169, ISSN: 0361-0470, DOI: 10.1094/ASBCJ-2008-0818-01**
- **GOUN E A ET AL: "Anticancer and antithrombin activity of Russian plants", JOURNAL OF ETHNOPHARMACOLOGY 2002 IE, vol. 81, no. 3, 2002, pages 337-342, XP002688318, ISSN: 0378-8741**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **GUO-QING HE ET AL: "Optimization of conditions for supercritical fluid extraction of flavonoids from hops ( Humulus lupulus L.)", JOURNAL OF ZHEJIANG UNIVERSITY SCIENCE, vol. 6B, no. 10, 1 October 2005 (2005-10-01), pages 999-1004, XP055045718, ISSN: 1009-3095, DOI: 10.1631/jzus.2005.B0999**

**Description**

FIELD OF THE INVENTION

The present invention relates to polyphenolic formulation suitable for use in modulation of the platelet activation processes.

BACKGROUND OF THE INVENTION

It is well known that vascular endothelium is involved in the modulation of platelet function. Endothelium is a metabolically active layer of cells lining blood vessels, and specialized in the regulation of vascular permeability, vascular tension, proinflammatory cell adhesion, coagulation, and platelet aggregation. Maintaining the functional integrity of vascular endothelium protects the vessels, and avoids the formation of atherosclerotic lesions. Modern therapeutic strategies are focused on protecting vascular endothelium function, particularly on maintaining its activity, which reduce the risk of cardiovascular diseases. Thereby, interfering with the biological activity of vascular endothelium in given pathophysiological states, it is possible to indirectly mitigate some of the risk factors for cardiovascular diseases.

Vascular endothelium has at least 3 mechanisms that protect platelets against activation. The main mediator released by vascular endothelium cells is nitric oxide (NO), which, because of its importance, is often considered to be a marker of *endothelium* normal activity. This simple chemical compound not only reduces platelet reactivity (adhesion, and aggregation), but also reduces the adhesion of leukocytes to vascular endothelium surfaces, and inhibits proliferation of vascular muscle cells. Generation of NO by endothelial nitric oxide synthase (eNOS) is regulated by multiple molecular mechanisms, which include, among others, regulation of eNOS gene expression, and enzymatic activity of eNOS. eNOS activity and its intracellular localization are strictly regulated by the co-, and post-translational modifications (phosphorylation of numerous amino acid residues of the enzyme, mainly serine residues, S-nitrosylation, acylation), as well as by protein-protein interactions. Phosphorylation of serine at position 1177 (Ser1177), and dephosphorylation of threonine 495 (Thr495) are the major modifications regulating the activity of eNOS. The literature describes an increased generation of NO under the influence of polyphenols contained in red wine through stimulation of eNOS expression, and activity.

In the modulation of platelet function, in addition to nitric oxide and prostacyclin (PGI2) released by endothelium, one can also mention endothelial ecto-nucleotidase type 1 (CD39; NTPDase1). CD39, like CD73 (5'-nucleotidase), is a transmembrane protein, located in caveolae, or invaginations of cell membrane in various cells. In addition to endothelium, CD39 is present on monocytes, vascular smooth muscle cells, platelets, and erythrocytes. Analysis of CD39 expression in vascular endothelial cells, platelets, and leukocytes demonstrated a correlation between CD39 expression, and its activity. The mechanism of action for NO, PGI2, and CD39 is related to the ATP / ADP metabolism. Thanks to NO, and PGI2, there is an interplatelet increase of cAMP, and cGMP, which results in the inhibition of platelet aggregation. Activity of CD39 is dependent on the presence of divalent ions ($Mg^{++}$/ $Ca^{++}$) and consists of hydrolyzing ATP, or ADP released from platelets to AMP, which then, in the presence of CD73, is converted into adenosine. Adenosine acts as an endogenous platelet inhibitor, which, by binding to the platelet $A_{2A}$ receptor, reduces platelet reactivity. It is believed that the protective function of CD39 comes down both to inhibiting the activation of platelets, and neutrophils, and to preventing the formation of blood clots by keeping the appropriate level of concentration of adenosine nucleotides. In this perspective, the interest of researchers in the studies on potential therapeutic application of CD39 in antiplatelet, and anticoagulation therapy, is not surprising. In the inflammatory conditions (vascular endothelium activation), a decrease in CD39 activity is observed. Similarly, changes in cholesterol homeostasis adversely affect the activity of CD39. It is believed that thrombin, which is generated *in vivo* in greater amounts in patients with hypercholesterolemia, changes the anticoagulant properties of vascular endothelium (reduces the activity of endothelial CD39) through the mechanism of activation of the transcription factor NF-$\kappa$B via endothelial receptors PAR. Apart from thrombin, also cytokines (TNF$\alpha$) can decrease CD39 expression and its activation. Literature data indicate that both epigallocatechin gallate contained in green tea, as well as resveratrol, and quercetin found in grapes, actively participate in reducing the proinflammatory and prothrombotic potential with the participation of CD39-dependent mechanism.

Processes associated with the activation of platelets play a major role in patients with diabetes. Cardiovascular complications are the most important cause for increased mortality in patients with diabetes. There is a constant need for new formulations exhibiting a protective effect on the cardiovascular system in the state of persistent hyperglycaemia.

Also important in the treatment or prophylaxis against cardiovascular diseases is modulation of processes linked with platelet aggregation and coagulation.

Within the trend of developing modern therapeutic strategies related to the protection of cardiovascular system, attention has been drawn to the use of plant polyphenols. Diverse chemical, physical and biological properties, as well as multidirectional biological activity of polyphenols is the result of a very diversified structure of polyphenolic compounds. These compounds have been shown to exhibit antioxidant, anti-inflammatory, antiallergic, antihepatotoxic, antimutagenic, anticancer, and antiatherosclerotic activity.

Due to the multidirectional protective effects of polyphenols on cardiovascular system, developing a new prophylaxis, and a treatment strategy for cardiovascular diseases with the use of plant polyphenols is more and more often considered.

The group of polyphenols exhibiting bioactivity includes prenylated flavonoids, present in hops extracts, and in particular chalcones, ex. xanthohumol, and desmethylxanthohumol. Desmethylxanthohumol is a precursor of 8-prenylnaringenin, exhibiting stronger estrogenic activity than other known phytoestrogens. On the other hand, xanthohumol is a precursor of isoxanthohumol, which exhibits antiproliferative activity with an extremely broad spectrum of mechanisms of inhibition at the stage of initiation, promotion, and progression of carcinogenesis.

The use of biological activity of chalcones contained in hops became the subject of research, the research being focused on how to obtain methods enabling to increase the number of prenylated flavonoids contained in hops extracts, or to develop methods allowing to obtain specific compositions of specific prenylated flavonoids exhibiting the desired properties.

Patent application EP1543834 discloses a method of producing hops extracts with an increased number of prenylated flavonoids, or their derivatives exhibiting estrogenic, and anti-proliferative activity. In the disclosed method, the hops product is subjected to isomerisation in the presence of a base, which contributes to the increase of 8-prenylnaringenin amount in the product, providing a multiple increase in estrogenic activity of the formulation obtained according to this method. At the same time, due to the proliferative properties of 8-prenylargenin, the applicant stressed the importance of adequate excess of xanthohumol in the composition, obtained according to the disclosed method.

U.S. patent application US2005/0042318 discloses a hops extract containing an increased amount of prenylated chalcones, which is used in the treatment of, or prophylaxis against diseases associated with estrogen deficiency.

The method for obtaining the active compounds with estrogenic properties from hops cones has been disclosed in WO83 00701 A1. Extract, with the use of carbon dioxide, from hops cones is treated with water, being an azeotropic agent, and then estrogenic active compounds are obtained by means of extraction with the use of ether, or chromatographic methods.

German patent application EP 199 39 350 A1 discloses a hops extract characterized by an increased xanthohumol content, the amount of prenylnaringenin in the extract being unspecified. The indicated use of the extract constitutes a supplement to beer and fruit juices.

WO2009/023710 describes a xanthohumol enriched hop extract which can be used for treating oxidative stress-associated medical conditions, such as cancer (e.g., breast, prostate, colon, and ovarian), aging, atherosclerosis, ischemic injury, inflammation, and neurodegenerative diseases (e.g., Parkinson's and Alzheimer's). US 2009/0291071 discloses dried component obtained from a beer brewing waste stream, wherein said component contains meaningful levels of xanthohumol. Said evaporated beer component was shown to inhibit various metabolic reactions such as oxidation and glycation of lipoproteins, including LDLs, and thereby contributing to reduction of atherosclerosis.

Although the disclosures of the prior art indicate beneficial properties of prenylated flavonoids contained in hops extracts, then, at the same time, the prior art shows that known hops extracts, due to the proliferative properties of 8-prenylnaringenin, must contain an excess of appropriate antiproliferative component, such as a xanthohumol. At the same time, the content of lipophilic compounds in the extract hinders lyophilisation of formulations of this type.

## BRIEF SUMMARY OF THE INVENTION

The objective of the present invention is to develop a novel polyphenolic formulation for use in the production of pharmaceutical agents applicable in the treatment of, and prophylaxis against cardiovascular diseases, and for use in nutritional supplements ensuring protective effects on the cardiovascular system.

These and other objectives as ensuing from the following description are attained by the subject matter of independent claims 1 and 7 while dependent claims refer to certain preferred embodiments of the present invention.

Generally, the present invention concerns a polyphenolic formulation having antithrombotic activity and containing defatted extract from spent hops.

Present disclosure provides a process for preparing a polyphenolic formulation having antithrombotic activity, in which process the fragmented spent hops are subjected to at least single extraction with an organic solvent, preferably at room temperature, the obtained extract is filtered and followed by removal of the organic solvent, wherein lipophilic compounds are removed from the resulting aqueous extract, and possibly the obtained extract is then concentrated and lyophilized.

In the process acetone is used as an organic solvent, preferably 70% aqueous acetone solution, whereas said lipophilic compounds are removed by means of extraction at the preferred volume ration of 1:1 with non-polar solvent, preferably chloroform, more preferably dichloromethane.

The invention relates to a pharmaceutical agent having antithrombotic activity, containing an active substance, and possibly additives, wherein said pharmaceutical agent contains defatted extract from spent hops as an active substance.

In another aspect, the invention provides nutritional supplement, for human or animals containing defatted extract from spent hops.

Further object of the invention relates to the use of defatted extract from spent hops for the manufacture of the medicament for the antithrombotic treatment or antithrombotic prophylaxis in human or animals subjects.

In certain preferred embodiment the invention relates to the use of defatted extract from spent hops for the manufacture of the medicament for antithrombotic treatment or antithrombotic prophylaxis in human or animals, wherein said treatment or prophylaxis against said diseases is associated with antiplatelet action.

In another preferred embodiment the invention relates to the use of defatted extract from spent hops for the manufacture of the medicament for antithrombotic treatment or antothrombotic prophylaxis in human or animals subjects, wherein said treatment or prophylaxis against said diseases is associated with anticoagulant action.

In further preferred embodiment, the invention relates to the use of defatted extract from spent hops for the manufacture of the medicament for antithrombotic treatment or antothrombotic prophylaxis in human or animals subjects, wherein said treatment or prophylaxis against said diseases is associated with the modulation of vascular endothelium activity.

In further preferred embodiment the present invention provides the use of defatted extract from spent hops for the manufacture of the medicament for antithrombotic treatment or antithrombotic prophylaxis of cardiovascular diseases in human or animals subjects.

In further preferred embodiment the present invention relates to the use of defatted extract from spent hops for the manufacture of the medicament for antithrombotic treatment or antithrombotic prophylaxis of atherosclerosis, ischemic heart disease, aorta and peripheral vascular diseases, arterial thrombosis, peripheral vein disease, venous thromboembolism in human or animals subjects.

In further embodiment, the present invention relates to the use of defatted extract from spent hops for the manufacture of the medicament for the cardiovascular protective treatment in the state of persistent hyperglycaemia in human or animal subjects.

In further embodiment, the present invention relates to the use of defatted extract from spent hops in the manufacture of a nutritional supplement intended for human or animals.

## BRIEF DESCRIPTION OF THE DRAWINGS

Fig. 1 shows the calibration curve for determining polyphenols using the Folin-Ciocalteu method.
Fig. 2 shows the calibration curve for determining catechin content.
Fig. 3 shows the survival curves of animals with diabetes, supplemented with the polyphenolic formulation.
Fig. 4 shows the change in body weight of animals with diabetes, supplemented with the polyphenolic formulation.
Fig. 5 shows the data on glycaemia in animals with diabetes, supplemented with the polyphenolic formulation.

## DETAILED DESCRIPTION OF THE INVENTION

### Definitions

The term "spent hops" refers to a solid waste product, formed during production of hops oil, used in brewing as a result of extraction of hops cones. Spent hops is a troublesome, powdered waste, disposal of which is a major problem due to the environmental contamination. This is a kind of waste that cannot be discharged into the sewer system as sewage, because it makes the sewers blocked.

As used herein, the term "defatted extract from hops" refers to an extract, from which the lipophilic compounds have been removed, including prenylated flavonoids.

The term "formulation having antithrombotic activity"" as used herein refers to the formulation which inhibits formation of arterial and venous thrombosis. Inhibition of arterial and venous thrombosis can achieved by formulation which modulates the activity of vascular endothelium, formulation exhibiting antiplatelet activity, formulation exhibiting anticoagulation activity, said activities being further defined in the ensuing description.

As used herein the term "formulation modulating the activity of vascular endothelium" refers to a formulation exerting a beneficial effect on the functioning of vascular endothelium cells, thus, contributing to the increased expression of CD39/NTPDase1 and the secretion of chemical substances, such as nitric oxide (NO). The beneficial effect of modulating the activity of vascular endothelium cells can be used in the treatment of, and prophylaxis against diseases, such as atherosclerosis, ischemic heart disease, aorta and peripheral vascular diseases, peripheral vein disease, venous thromboembolism.

The term "formulation exhibiting antiplatelet activity" refers to a formulation inhibiting platelet reactivity. Known

in the prior art methods for determining the antiplatelet activity of the formulation are a method of turbidimetric aggregation or impedance aggregation.

The term "formulation exhibiting anticoagulation activity" refers to formulation inhibiting blood coagulation. Known in the prior art methods for determining the anticoagulant activity of the formulation are the following methods: prothrombin time (PT), thrombin time (TT),.

The method for obtaining the formulation according to the invention consists in the extraction of polyphenols from hops waste by means of at least a single extraction using an organic solvent. A suitable solvent is selected from a group of alcohols and their aqueous solutions, ketones, and their aqueous solutions, with or without the addition of inorganic or organic acid, and mixtures of these solvents. The preferred solvent in the method according to the invention is a solvent of medium polarity, such as, ex. acetone. In the case of extraction of hops waste, the use of triple extraction of 70% aqueous acetone at room temperature, using a 10-times the excess of extractant in relation to the amount of substrate, turned out to be preferable from the process efficiency viewpoint. Extraction was conducted with stirring, and then extracts were filtered and concentrated so as to remove the organic solvent.

From the extract obtained this way, the lipophilic compounds are removed. This can be achieved for example by means of extraction with a solvent, such as chloroform. The defatted aqueous extract is then concentrated in order to remove any residual solvent. Good results can also be achieved by using other non-polar solvents, such as hexane or dichlormethane, instead of chloroform. It has been demonstrated that removal of the lipophilic compounds results in higher antiplatelet activity of the formulation.

The formulation can be possibly subjected to lyophilisation, and stored in the dark at 4° C, in airtight containers. The total polyphenols content equals approximately 240 mg / g of formulation in terms of gallic acid. By the total polyphenols content in terms of gallic acid content it is meant a content determined in accordance with the Folin-Ciocalteu method for determination of polyphenolic compounds (Bordonaba, and Terry 2008).

The studies performed on the formulation regarding its effect on the expression of CD39 on the surface of human vascular endothelium cells demonstrated that the formulation according to the invention increases the level of expression of CD39 on the cell surface, this effect depending on the concentration of the formulation. A significant increase in expression was observed for concentration of 6 μg / ml in terms of gallic acid, the effect of the formulation continuing for the next 24 hours despite the removal of the formulation from the cell medium.

Also, the study on the activity of nitric oxide synthase (eNOS) confirms the influence of the formulation according to the invention on the vascular endothelium activity. The results obtained from measuring the level of phosphorylation of eNOS indicate a significant influence of the formulation according to the invention on the modulation of the amino acids phosphorylation in protein, which indicates an increase in the activity of nitric oxide synthase, and, consequently, an increase in the NO production by vascular endothelial cells.

The obtained results are further confirmed by studies on platelet interaction with vascular endothelial cells subjected to exposure to the formulation according to the invention. Assessment of the expression of surface markers of platelet activation (CD62, and PAC1) indicates a reduction in expression of PAC-1 (active form of a fibrinogen receptor GPIIb-IIIa) on the surface of platelets incubated with endothelium treated with formulation according to the invention, which confirms the effects of the formulation according to the invention on the expression of CD39, and the activity of eNOS.

The obtained results confirm the beneficial effect of the formulation according to the invention on the activity of vascular endothelium. Formulation according to the invention can also be used as a pharmaceutical active substance having a modulating effect on the vascular endothelium activity.

Study of platelet aggregation by impedance and optical aggregometry of aqueous solutions of polyphenolic formulation lyophilisate, conducted in both healthy volunteers, and patients receiving prophylactically an acetylsalicylic acid (ASA), demonstrated high antiplatelet activity of the formulation in both studied groups, but greater activity was observed in patients receiving ASA. The formulation may therefore find use in the treatment of, and prophylaxis against diseases whose treatment and prophylaxis are associated with influence on platelet aggregation. Demonstrated antiplatelet activity of the formulation according to the invention with respect to aggregation induced by adenosine diphosphate (ADP) constitutes a very advantageous property of the formulation. This activity profile makes it possible to use the formulation as an adjuvant in a classic antiplatelet therapy with low doses of acetylsalicylic acid in patients with ischemic heart disease. Formulation concentration in blood / plasma exhibiting the desired antiplatelet properties amounts to 7.5 - 15 μg / ml in terms of gallic acid. In individual cases, however, the dose may be accordingly adjusted by a person skilled in the art to the amount corresponding to a specific application, Formulation according to the invention can thus be used as an active substance in pharmaceutical agents exhibiting an antiplatelet effect.

The result of the studies conducted using thromboelastometry also confirmed the anticoagulant properties of the formulation according to the invention, which can thus be used as an active substance in pharmaceutical agents exhibiting an anticoagulant effect.

Further studies conducted with the formulation according to the invention have demonstrated that the administration of the formulation at doses of 2.5 and 5 mg / kg bw / day in terms of gallic acid provides significantly longer

survival in animals suffering from diabetes, while not adversely affecting the condition of the animals. Formulation dose exhibiting the desirable protective properties of the formulation is 5 mg / kg bw / day in terms of gallic acid. In individual cases, however, the dose may be adjusted by a person skilled in the art to the amount corresponding to a specific application, The formulation according to the invention can thus be used as an active substance of pharmaceutical agents supporting the treatment or the prophylaxis against diabetes.

It is allowed to combine the formulation with the known in the art and conventionally used in practice pharmaceutical additives, the formulation can be subjected to the known methods enabling pharmaceutical compositions to have a solid or liquid form, that can be administered orally or parenterally. Multiparameter study of cytotoxicity also showed that the formulation according to the invention is not a cytotoxic substance. The conducted study of cytotoxicity of the formulation indicate that the formulations according to the invention containing the total amount of polyphenols of less than 30 ug / ml of the prepared solution do not exhibit cytotoxicity.

The positive antithrombotic activity, demonstrated by the use of the formulation according to present invention, makes the formulation attractive for the production of nutritional supplements (dietary supplements), in particular intended for administration to patients with risk of cardiovascular diseases according to SCORE (Systemic Coronary Risk Evalualtion)> 5% (high risk patients), and <10% (high risk patients with indications to use ASA within the primary prevention). By SCORE, the SCORE risk scale is meant. It is a tool for assessing an individual risk of death from cardiovascular disease over the next 10 years based on risk factors occurring in a given person. The scale was developed by the European Society of Cardiology.

In case of the use of the formulation according to the invention in the production of nutritional supplement, it is possible to add, known in the food industry, auxiliary materials, their properties and possible choice in order to produce the appropriate composition being well described in the literature and do not go beyond the routine operation of a person skilled in the art.

Apart from the advantageous properties related to antithrombotic activity, which the formulation demonstrates, the removal of the lipophilic compounds, including prenylargenin which has proliferative and estrogenic properties, adds to the safety of the formulation. Another advantage is the simplicity of the process to obtain the formulation, easy availability and low cost of basic raw material, being indeed an inconvenient waste material.

EXAMPLES

Example 1

**1.1. Preparation of polyphenolic formulation from hops waste**

The excess of fragmented hops waste was subjected to the process of triple extraction with the 70% solution of acetone at room temperature for 30 minutes (I extraction), and 15 minutes (II, and III extraction). Each time, 10-tuples excess of extractant in relation to the weight of raw material (v/w). The extraction was performed using a magnetic stirrer. The obtained extracts were filtered. In the next stage, the combined extracts, were concentrated 6 times in a vacuum evaporator at 40° C in order to remove the organic solvent. The obtained aqueous extract was then extracted 4 times with chloroform (1:1, v/v) in order to remove lipophilic compounds. The defatted aqueous extract was then about 2 times concentrated in order to remove any residual chloroform, and was lyophilized. The fragmented solid polyphenolic formulation, until analysis, was stored in sealed glass vessels at 4° C in the dark.

**1.2. Preparation of the stock solution**

In order to analyse the obtained polyphenolic formulation, stock solution for analysis was prepared as follows: 20 mg of polyphenolic formulation was weighed and dissolved in 10 $cm^3$ of 10% aqueous dimethyl sulfoxide (DMSO), and then the samples were placed for 5 min in an ultrasonic bath. The solution obtained this way was analysed to determine the content of polyphenolic compounds.

**1.3. Determination of the content of polyphenolic compounds using Folina-Ciocalteu method** [Bordonaba, and Terry, 2008]

This method is based on the reduction properties of polyphenolic compounds. Folin-Ciocalteu reagent contains a complex of tungsten-molybdenum compounds ($H_3PW_{12}O_{40}$ and $H_3PMo_{12}O_{40}$), which during the oxidation of polyphenols change into blue coloured forms of oxygen ($W_8O_{23}$ and $Mo_8O_{23}$), exhibiting maximum absorbance at 760 nm.

The intensity of colour is proportional to the amount present in the solution of phenolic substances.

$Na_2WO_4/Na_2MoO_4 \rightarrow ((\text{poliphenol}) - MoW_{11}O_{40})^{-4}$

$Mo(VI)\ (\text{yellow}) + \bar{e} \rightarrow Mo(V)\ (\text{blue})$

Determination was made as follows:

The proper assay:

10 $cm^3$ of distilled water,
0.2 - 2 $cm^3$ of solution of the studied assay,
0.5 $cm^3$ of Folina-Ciocalteu reagent,
5 $cm^3$ 20% $Na_2CO_3$.

As a whole, it was being completed with water up to 50 $cm^3$.
The reference probe did not include the studied sample. After 20 min, the absorbance of the proper probe was measured in relation to the reference probeat a wavelength of 760 nm. Polyphenol content was determined on the basis of the calibration curve shown in Fig. 1, which was made using methanol solution of gallic acid at a concentration of 0.2 mg / $cm^3$.

**1.4. Determining the flavanol content using the vanilin method [Swain-Hillis, 1959]**

The basic formulation obtained according to the basic point 2 of Example 1 was studied with a view to the flavanol content according to methodology described below.
Vanilin method is based on the reaction of vanillin with the catechins monomers, and polymers. The characteristic red colour is stable in the environment of 70% $H_2SO_4$, and exhibits the maximum of absorption at $\lambda$ = 500 nm.

Performing the determination:

The following probes were prepared:

**a -** 0.2-0.4 $cm^3$ of the studied basic solution diluted 5 times, complimented with distilled water up to 2 $cm^3$ and 4 $cm^3$ of vanilin reagent was added (1 g of vanilin in 100 $cm^3$ of 70% $H_2SO_4$),
**b -** 0.2-0.4 $cm^3$ of the studied basic solution diluted 5 times, complimented with distilled water up to 2 $cm^3$ and 4 $cm_3$ of 70% $H_2$SO4,
**c -** to 2$cm^3$ of distilled water, 4$cm^3$ of vanilin reagent was added,
**d -** to 2$cm^3$ of distilled water, 4$cm^3$ of 70% $H2_{SO}4$ was added,

Probes were placed in a cold water bath so that their temperature did not exceed 35° C After 15 minutes, the absorbance of probes a, b, c, d was measured with respect to d blank at $\lambda$=500 nm. From the calibration curve, the catechin concentration was read in the sample, corresponding to the absorbance $A = A_a - (A_b+A_c)$.
To prepare the calibration curve, an aqueous solution of (+) catechin at a concentration of 0.05 mg/$cm^3$ was used. The curve of absorbance relation $A = A_a - (A_b+A_c)$ as a function of the concentration of (+) catechin is shown in Fig. 2.

**1.5. Determination of proanthocyanidins [Rösch *et al.,* 2003]**

The basic formulation obtained according to the basic point 2 of Example 1 was studied with a view to the proanthocyanidins presence according to method described below. Procyanidins heated at 100° C in the environment of butanol acidified with hydrochloric acid undergo depolymerisation, and oxidation to the appropriate coloured anthocy-anidins.

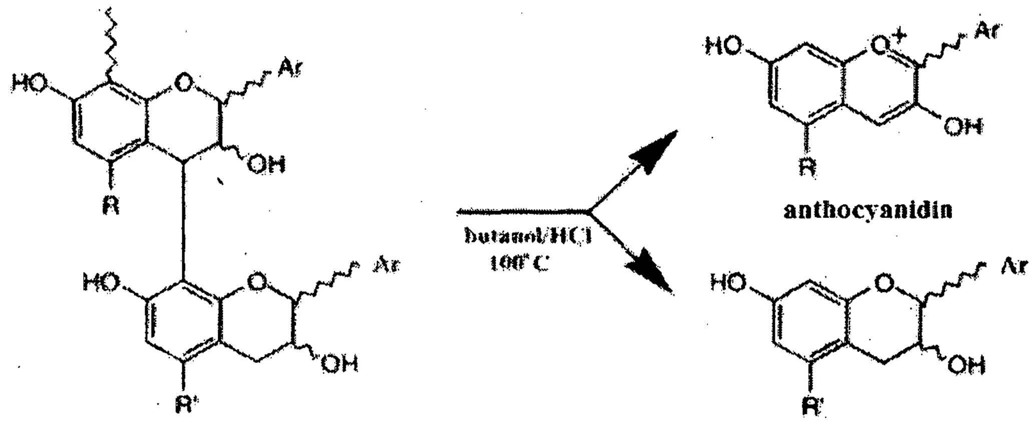


proanthocyanidin

Performing the determination:

To 0.2-0.4 $cm^3$ of the basic solution completed with up to 0.4 $cm^3$ of 10% DMSO, 9,6 $cm^3$ of mixture containing butanol, and concentrated hydrochloric acid (9:1, v/v) was added. Then, the samples were heated for 90 min in a boiling water bath. After cooling, the samples were filtered, completed to the volume of 10 $cm^3$ and the absorbance (A) at a wavelength $\lambda$ = 550 nm, in relation to the mixture of butanol / HCl, was measured. The proanthocyanidins content was converted into cyanidin, using the molar absorption coefficient $\varepsilon$ = 17360 $dm^3$/mol*cm.
Cyanidin concentration in the reaction mixture was calculated from the relation:

$$C_{cyanidin} = 0.0165\ A\ [mg/cm^3]$$

**1.6. Characteristics of polyphenols in the obtained formulations from hops waste**

Table 1 contains data from content determinations of 9 formulations obtained from hops waste by means of the method according to the invention. The table shows the total polyphenol content (determined according to point 3 of this example), flavanols content (determined according to point 4 of this example), and proanthocyanidins content determined according to point 5 of this example).

Table 1. The polyphenols content in the hops waste formulation

| Formulation No. | Content (mg/g of formulation) | | |
|---|---|---|---|
| | Polyphenols in total | Flavonols | Proanthocyanidins |
| 1 | 232.99 ± 21.62 | 119.83 ± 5.78 | 93.40 ± 4.37 |
| 2 | 244.83 ± 15.34 | 121.41 ± 2.64 | 87.82 ± 4.17 |
| 3 | 247.24 ± 14.63 | 126.10 ± 5.25 | 98.47 ± 3.04 |
| 4 | 198.50 ± 10.33 | 85.18 ± 3.20 | 81.09 ± 3.19 |
| 5 | 229.55 ± 5.67 | 100.10 ± 3.67 | 96.64 ± 7.16 |
| 6 | 242.59 ± 13.38 | 123.01 ± 7.06 | 95.91 ± 6.73 |
| 7 | 258.07 ± 11.03 | 134.85 ± 3.42 | 99.23 ± 8.07 |
| 8 | 252.82 ± 17.26 | 121.01 ± 5.85 | 87.11 ± 2.20 |
| 9 | 248.41 ± 2.94 | 127.09 ± 5.08 | 106.03 ± 3.45 |
| average | 239.44 ± 17.75 | 117.62 ± 15.32 | 93.97 ± 7.54 |

Example 2

**Modulation of vascular endothelium activity**

**2.1. Preparation of the studied polyphenolic formulations for studying function of vascular endothelium activity.**

The study included an extract obtained according to the method described in part 1 of example 1. The studied formulation containing the extract from hops waste was labelled in the present example as EO4. For each study cycle, a working solution was prepared based on a solid polyphenolic formulation obtained according to the procedure described in point 1 of example 1, with a concentration, in terms of gallic acid, 5 mg / ml in 10% DMSO. The obtained solution was filtered on a 0.22 μm sterile filters intended for filtration of aggressive liquids. After filtration, on the basis of the sterile stock solution of 5 mg / ml solutions, corresponding intermediate solutions of the formulation were prepared, which were added in the appropriate volume to the vascular endothelial cell cultures, to obtain final concentrations of 1 and 6 μg / ml.

2.2. The study material

The study material consisted of human vascular endothelial cells isolated from umbilical vein (HUVEC, Human Umbilical Vascular Endothelium Cells) and human platelets isolated from blood of healthy donors.

2.3. Methodology of the study

The study was carried out according to standard methods known in the art. For determination of CD39 expression on endothelial cell surface, and CD62, and PAC-1 on platelet surface, the phosphorylation of Ser1177, and Thr495, in eNOS (FlexSet), a flow cytometry was used. Colorimetric methods were used for the determination of the protein with the BCA method.

Exposure of endothelial cells to the polyphenolic formulation E04 at concentrations of 1, or 6 μg / ml amounted to 24 hours. Control system consisted of endothelial cells not treated with EO4.

Statistical analysis of the results

For statistical analysis, t test for paired samples, or test for a single trial with Bonferroni correction were used for multiple comparisons.

3.3. Results

1/ Expression of CD39 on the surface of human vascular endothelial cells

In order to check the influence of the polyphenolic formulation EO4 on the expression of CD39 on the surface of vascular endothelium cells, 11 independent experiments were carried out. In the experiment, the cells between the 3rd, and 7th passages were involved. The average level of expression of CD39 on the surface of control cells (no treated with E04) amounted to 29.0 ± 5.8% (percentage of CD39-positive cells).

It was observed that the E04 formulation increases the level of expression of CD39 on the cell surface, and,this effect depends on the concentration of the formulation. A significant increase of 50% was noted for the concentration of 6 μg / ml EO4 compared to the cells non-incubated with polyphenol ($p<0.01$).

The performed study allowed to conclude that the EO4 polyphenolic formulation increases the expression of CD39 (NTPDases) on the surface of vascular endothelial cells after 24 hours of incubation with the E04 formulation, and this effect continues for over the next 24 hours despite the removal of EO4 from the cell medium.

The obtained results indicate that the E04 formulation may contribute to the increased antiplatelet activity of endothelial cells, which, through the expression of CD39 on their surface, cause hydrolysis of ADP, a primary platelet stimulator. 2/ Activity of nitric oxide synthase (eNOS) - determination of phosphorylation of Ser1177, and Thr 495

Determination of the degree of phosphorylation of Ser1177, and Thr495 was performed in lysates of endothelial cells exposed to the activity of EO4 at concentrations of 0, 1, and 6 μg / ml for 24 hours. 10 experiments were carried out. For determinations, the method for monitoring the level of phosphorylation of eNOS in flow cytometer (Phospho eNOS (S1177), and Phospho eNOS (T495) Flex Set) was used.

There was a significant increase in the level of phosphorylation of Ser1177 after the activity of EO4 for both tested concentrations, compared to the cells not treated with E04 (254.6 ± 15.6 for 1 μg / ml, and 328.2 ± 20.4 for the concentration of 6 μg / ml vs. 221.4 ± 16.4 for the control, $p<0.01$). At the same time, a significant reduction in the degree of phosphorylation for Thr495 under influence of EO4 (255.9 ± 46.4 in the control, 196.8 ± 30.8 for the concentration of 1 ug / ml, and 168.1 ± 27.8 for the concentration of 6 μg / ml) was noted. Thus, the results obtained from measuring the level of phosphorylation of eNOS indicate a significant impact of the EO4 formulation on the modulation of the phosphorylation of amino acids in the protein, and, consequently, the increased eNOS activity, and an increase in NO production by endothelial cells.

3/ Assessment of platelet interaction with endothelial cells exposed to EO4

The experiment assessed the expression of the surface markers of platelet activation (CD62, PAC-1) using flow cytometry. Platelets were incubated with endothelial cells previously exposed to the activity of EO4 at concentrations of 1, and 6 μg / ml. Platelets in the presence of endothelial cells were stimulated with 10 μM of ADP. The control was constituted by the cells not treated with EO4.

A significant reduction in expression of PAC-1 (active form of the fibrinogen receptor, GPIIbIIIa) on the surface of platelets incubated with endothelium subjected to the activity of EO4 at the concentration of 6 μg / ml compared to the control ($p<0.01$).

The obtained results confirm the earlier observations regarding the effect of EO4 on the expression of CD39 EO4 (a significant increase in CD39 expression), and on the activity of eNOS (increase in Ser1177 phosphorylation, and decrease in Thr495 phosphorylation).

Example 3

**Antiplatelet activity**

**3.1. Preparation of the studied polyphenolic formulations.**

The study included extracts obtained from two independent preparations carried out in accordance with the method described in part 1 of example 1. The studied formulations from spent hops indicated in the present example as EO4(1), and EO4(2). For each study cycle, a working solution of polyphenolic formulation EO4 at concentration, in terms of gallic acid, 1.5 mg/ml in 10% DMSO, was prepared. The solution was prepared according to the protocol shown below:

- 7.5 mg of lyophilized polyphenolic extract was dissolved in 1000 μl of previously prepared solution (PBS without Ca, and Mg, with the addition of DMSO to the final concentration of 10%). Solution prepared this way constituted a stock solution.
- stock solution was then agitated for 1 min. (850 rpm, 37° C) and 15 min. (500 rpm, 37° C)
- solution was then centrifuged (5 min., 9200 g)
- in the presence of sediment, supernatant was removed for further tests
- solutions, in which there was no sediment, and the supernatants were diluted so that the final concentration of gallic acid in solutions amounted to 1.5 mg / ml

**3.2. The study population**

Study on the impact of polyphenolic formulations on platelet function were conducted in healthy volunteers (Group 1), and patients with cardiovascular disease, after surgical treatment of ischemic heart disease, chronically treated with acetylsalicylic acid (ASA) at a dose of 150, or 75 mg of ASA per day (Group 2). This approach and division of work between the Groups have substantive justification (potential differences in responses between healthy donors, and patients on ASA therapy). The use of the same procedures, and protocols by both Groups (except for the study of the effect of polyphenols on the aggregation induced by arachidonic acid (AA) in patients taking aspirin) was assumed.

Blood was collected using Sarstedt's sets. Anticoagulant was selected according to the method of analysis of platelet function. In the study of aggregation by the optical method, 3.2% buffered sodium citrate was used. In the study of aggregation by the impedance method - Refludan ® hirudin (25 ug/ml). In both cases, the blood : anticoagulant rate of 9:1 was maintained.

Blood collection was preceded by a written consent for the study (having explained the purpose, and nature of the study). The experiment was covered by the consent of the Commission of Bioethics, Medical University in łódź, no RNN/13/07/KB, dated 16.1.2007.

**3.3.** Materials and methods

Blood samples from healthy donors, and patients after coronary interventions taking ASA for a long time were

both subjected to the study of polyphenolic formulation effects. The applied methodology for the study constituted stratified randomization study (drawing tube positions in aggregometer for a predefined panel of concentrations).

Both for the impedance method and the turbidimetric method, the parameter defined as a result of aggregation (Aggregation Score - AS) was used as a unit of observation.

$$AS = \text{maximum aggregation (Amax) x area under the curve (AUC)}$$

Estimation of sample size was based on maximum aggregation parameter (Amax), assuming:

Significance level = 0.05
Power of test = 0.8

Student's t-test for dependent assays was used.
The determinations were made in paired-wise manner(measurements before and after administration of test compound). Inhibitory efficacy of flavonoids is low, and was estimated at 10%. Measurement variability of 10% was assumed. Following the adoption of such conditions, the minimum number of experiments amounts to 16 individuals for each studied formulation (16 healthy donors, and 16 patients with cardiovascular diseases).

**3.4. Methods of platelet aggregation study**

**A/ Platelet aggregation study by optical aggregometry method (Light Transmission Aggregation - LTA)**

Optical aggregation is regarded as a so-called gold standard among the methods of the study of platelet reactivity. It is based on monitoring and recording changes in the transmittance of light incident on the assay of platelet rich plasma, which occur during platelet aggregation in response to agonist.

Platelet rich plasma (PRP) was obtained by centrifugation of whole blood collected in sodium citrate as an anticoagulant (250xg, 6 min). The supernatant was removed and the residue was centrifuged again (2000xg, 10 min). PRP with platelet concentration of $200x10^9$/L was used in the study.

Concentrations of EO4 formulation in the assay amounted to 7.5 and 15 μg / ml in terms of gallic acid, 5, and 10 μmol / L ADP being used as an agonist. The incubation time of PRP with the formulation was 10 min (at 37 ° C). Measurements were made in a paired-wise manner. The control sample consisted of platelet rich plasma with an adequate amount of 10% DMSO. Platelet aggregation was monitored for 15 minutes, using an optical aggregometer Chrono-Log 490-2D (Chrono-Log, Havertown, PA USA)

**B/ Platelet aggregation study by impedance method - multiple electrode aggregometry (MEA)**

Multiplate apparatus (Dynabyte Medical) allows for the assessment of platelet function in whole blood based on the principle of impedance aggregometry. This method is based on monitoring the changes in impedance, which are associated with adhesion of platelet aggregates on the surface of electrodes. The device allows for conducting simultaneous measurements of five independent samples. Concentrations of E04 formulation in the studied assays were 7.5, and 15 μg / ml in terms of gallic acid. Blood with the extract was incubated 15 min before measurement at room temperature. Aggregation measurement was carried out simultaneously in three assays, one of which being the control, and the two - studied assays containing different amounts of polyphenolic formulation. As recommended by the producer, 6.4 μmol / L ADP, collagen at a concentration of 3.2 μg / ml, and arachidonic acid at a concentration of 0.5 μmol / L were used as an agonist. Measurement time was 15 min.

*Statistical analysis*

Data were presented as mean ± standard error, or as medians and quartile ranges. In order to verify the normality of data distribution, Shapiro-Wilk test was used. In a case of normal distribution of data, one-sided paired t-test was used with the Bonferroni correction. In a case of data with the distribution deviating from normal, Wilcoxon test was used. The results are presented in the following tables, where the results of aggregation (AS) for the different concentrations of formulations (Conc. E04), as well as the percentage of inhibition (% inh.), and the level of significance (p) are presented.

Table 3. The results of aggregation inhibition in whole blood (agonist: collagen)

| Group 1 (healthy volunteers) | | | | | | | |
|---|---|---|---|---|---|---|---|
| Agonist | AS (collagen 3.2/μg/ml) | | | | | | |
| EO4 Conc. | 0 | 7,5 | % inh. | p | 15 | % inh. | p |
| EO4(1) | 1155.7 ±181.0 | 1022.5 ±106.6 | 5.6 ±9.8 | 0.140 | 920.0 ±76.1 | 15.0 ± 6.1 | 0.038 |
| EO4(2) | 1155.7 ± 181.0 | 974.0 ± 101.9 | 10.1 ± 10.2 | 0.0942 | 998.8 ± 67.8 | 4.7 ± 11.6 | 0.166 |
| Group 2 (patients with cardiovascular diseases) | | | | | | | |
| EO4(1) | 610.1 ± 97.5 | 447.5 ± 57.8 | 19.0 ± 9.1 | 0.0182 | 380.9 ± 57.7 | 30.0 ± 10.6 | 0.003 |
| EO4(2) | 634.9 ± 104.9 | 570.8 ± 74.8 | 0.3 ± 9.8 | 0.3538 | 429.6 ± 61.9 | 15.1 ± 17.1 | 0.179 |

Table 4. The results of inhibition of platelet aggregation in whole blood (agonist: ADP)

| Group 1 (healthy volunteers) | | | | | | | |
|---|---|---|---|---|---|---|---|
| Agonist | AS (ADP 6.4 μM) | | | | | | |
| EO4 Conc. | 0 | 7.5 | % inh. | p | 15 | % inh. | p |
| EO4(1) | 558.2 ± 48.0 | 532.8 ±86.1 | 4.7 ±12.9 | 0.3588 | 571.7 ± 74.4 | -3.1 ± 11.2 | 0.4149 |
| EO4(2) | 558.2 48.0 | 548.8 ± 88.1 | 1.0 ± 15.1 | 0.4531 | 626.0 63.9 | -14.7 ± 11.6 | 0.1548 |
| Group 2 (patients) | | | | | | | |
| EO4(1) | 328.0 ± 107.6 | 181.7 ± 51.4 | 28.0 ± 14.7 | 0.0185 | 177. 4 ± 57.3 | 10.6 ± 23.3 | 0.076 |
| EO4(2) | 337.0 ± 110.2 | 176.9 ± 51.2 | 28.3 15.3 | 0.0185 | 120.0 ± 38.3 | 44.3 ± 13.3 | 0.0071 |

Table 5. The results of inhibition of aggregation in whole blood (arachidonic acid (AA) as an agonist - In the case of Group 2, AA was not used because it would be unjustified: patients take aspirin, which results in complete inhibition of aggregation induced bv arachidonic acid.

| Group 1 (healthy volunteers) | | | | | | | |
|---|---|---|---|---|---|---|---|
| Agonist | AS (AA 0.5 mM) | | | | | | |
| EO4 Conc. | 0 | 7.5 | % inh. | p | 15 | % inh. | p |
| EO4(1) | 652.5 56.1 | 708.1 ± 63.6 | -9.5 ± 6.5 | 0.1049 | 733.5 ±118.3 | -9.9 ± 8.9 | 0.19 |
| EO4(2) | 652.5 ± 56.1 | 694.8 ± 63.7 | -6.8 ± 4.8 | 0.1165 | 609.9 ± 41.2 | 5.3 ± 3.9 | 0.08 |

Table 6. Results of aggregation inhibition in platelet-rich plasma (agonist: ADP)

| **Group 1 (healthy volunteers)** | | | | | | | | |
|---|---|---|---|---|---|---|---|---|
| Agonist | **AS (ADP 5 μM)** | | | | | | | |
| EO4 Conc. | 0 | 7.5 | % inh. | p | 0 | 15 | % inh. | p |
| EO4(1) | 37.8 ± 5.9 | 32.4 ± 10.7 | 17.1 ± .19.9 | 0.2188 | 48.7 ± 10.3 | 35.9 ± 14.5 | 27.6 ± 32.5 | 0.2176 |
| EO4(2) | 50.3 ± 9.8 | 31.2 ± 10.3 | 41.4 ± 20.8 | 0.0592 | 48.7 ± 10.3 | 27.8 ± 9.7 | 46.1 ± 17.5 | 0.063 |

(continued)

| Group 2 (patients) | | | | | | | | |
|---|---|---|---|---|---|---|---|---|
| EO4(1) | 22.2 ± 4.1 | 6.4 ± 2.1 | 71.9 ± 5.4 | 0.0008 | 15.7 ± 3.6 | 7.8 ± 3.6 | 49.3 ± 25.3 | 0.065 |
| EO4(2) | 19.8 ± 3.9 | 8.8 ± 3.5 | 63.1 ± 11.2 | 0.002 | 17.1 ± 2.5 | 8.2 ± 2.7 | 54.1 ± 17.5 | 0.0113 |

Table 7. Results of aggregation inhibition in platelet-rich plasma (agonist: ADP)

| Group 1 (healthy volunteers) | | | | | | | | |
|---|---|---|---|---|---|---|---|---|
| Agonist | AS (ADP 10 μM) | | | | | | | |
| EO4 Conc. | 0 | 7.5 | % inh. | p | 0 | 15 | % inh. | p |
| EO4(1) | 51.8 ± 6.6 | 28.5 ± 9.3 | 50.8 ± 13.6 | 0.002 | 51.1 7.3 | 38.7 ± 13.2 | 27.8 ± 22.9 | 0.1704 |
| EO4(2) | 52.7 6.6 | 33.6 ± 9.6 | 39.1 ± 15.6 | 0.028 | 51.1 7.3 | 33.7 ± 10.0 | 36.9 ± 19.1 | 0.057 |
| **Group 2 (patients)** | | | | | | | | |
| EO4(1) | 40.5 7.9 | 12.0 ± 4.9 | I 70.7 ± 8.1 | 0.003 | 26.0 ± 6.4 | ± 4.6 | 16.3 ± 15.8 | 0.2272 |
| EO4(2) | 40.5 ± 7.9 | 12.1 ± 3.3 | 69.8 ± 7.4 | 0.003 | 28.3 ± 6.8 | 9.4 ± 1.9 | 59.3 ± 9.7 | 0.0195 |

Both tested formulations EO4 (1), and EO4 (2) exhibit similar, high antiplatelet activity, and a similar profile of activity - antiplatelet activity mainly relates to ADP-induced aggregation. The inhibitory effect of formulation on the ADP-induced aggregation is slightly higher in patients undergoing the treatment of ASA, compared to the group of healthy donors.

## Example 4

### Effect of removal of lipophilic compounds on antiplatelet properties of formulation according to present invention

### 4.1 Materials and methods

The procedure to obtain study samples followed that described in part 1 of example.

In addition to formulation obtained using chloroform to remove lipophilic compounds, an additional formulation was prepared wherein instead of chloroform, dichloromethane was used. Also prepared was an additional sample wherein no defatting stage (i.e. stage of removal of lipophilic compounds) was employed.

In effect the following samples were studied:

EO4 (0) - formulation without defatting
EO4 (Ch) - formulation defatted with chloroform
EO4 (DM) - formulation defatted with dichloromethane

The study was performed according to methodology described earlier in example 3.

### 4.2. Results

All studied formulations exhibited antiplatelet properties in both measurement systems (i.e. earlier described impedance aggregation and optical aggregation).

Table 8. The results of platelet aggregation in whole blood (impedance aggregation using collagen as an agonist) after incubation with formulations without the use of defatting, and after defatting with two solvents

| Studied group - patients with cardiovascular disease | | | | |
|---|---|---|---|---|
| Agonist | AS (collagen 4.2/μg/ml) | | | |
| EO4 concentration (μg/ml) | control | without defatting (0) | Chloroform (Ch) | Dichloromethane (DM) |
| 7.5 | 722.2 ± 90.1 | 621.4 ± 59.8 | 508.6 ± 86.3* | 415.1 ± 60.7** |

Results are presented in AC units (aggregation score) as means, and standard error.
For the concentration of 7.5 μg/ml

* EO4 (0) ≠ EO4 (Ch), $p<0.05$
** EO4 (0) ≠ EO4 (DM), $p<0.03$

As indicated by the results presented in the table 8, removal of lipophilic compounds resulted in higher antiplatelet activity with highest activity obtained for samples defatted using dichloromethane.

**Example 5**

**Anticoagulant effect of the formulation according to invention.**

Standard laboratory coagulation studies allow for the assessment of the complex impact of anticoagulant substances on: coagulation activation pathway dependent on a tissue factor and coagulation activation pathway dependent on contact factors.
Thromboelastometry allows for assessment of the complex impact of anticoagulant substances on platelet reactivity and activity of plasma factors. The demonstration of such a complex operation may give rise to a new generation of substances with both antiplatelet and anticoagulant properties.

**5.1 Materials and methods**

The procedure to obtain study samples followed that described in part 1 of example. In this example the obtained formulation is referred to as EO4.

5.1.1 Coagulation studies

In the study, an automatic coagulant analyzer Sysmex C-540, and Thromborel S, Pathromtin SL, and Thrombin Test reagents by Siemens (Siemens Healthcare Diagnostics Products GmbH, Marburg, Germany) were used.

Studied parameters

PT - prothrombin time was determined using Thromborel S reagent, by Quick's Method). The PT test is used in the examination of the efficiency of the coagulation activation pathway dependent on a tissue factor.
TT - thrombin time was determined using a Test Thrombin reagent.
The TT test is used in the examination of the efficiency of the coagulation activation common pathway.
APTT - activated partial thromboplastin time was determined using Pathromtin SL reagent. The APTT test is used in the examination of the efficiency of the pathway dependent on contact factors. The study was conducted according to the protocol of the assay producer.

5.1.2 Thromboelastometry method

In the study, ROTEM® thromboelastometer, by Pentapharm GmbH, Munich, Germany), and EO4 polyphenolic formulation were used. The analysis involved three assessment parameters of the thromboelastographic curve:

Studied parameters

CT- coagulation time, i.e. time required to form a clot with consistency of 2 mm, counted from the test start, or from the moment of adding an activator (reagents, and calcium ions).
$\alpha$ - angle alpha, defined as the angle of inclination of the tangent to the coagulation curve at the point of 2 mm. It defines the kinetics of clot formation. This parameter is given in degrees (o), and corresponds to an angle formed with a zero line.
CFT - clot formation time characterizes the next coagulation phase, the kinetics of formation of a stable clot, both from platelet, and from fibrin. CFT is defined as time which elapsed between clot consistency of 2 mm, and 20 mm.

Study has been performed according to the producer's procedure, with the use of original cuvettes, and the set for the rex-tem®test.

**5.2 Results**

EO4 formulation in a concentration of 15 μg /ml exhibited in vitro anticoagulant properties in the APTT study.

Table 9. APTT test results after incubation of the healthy volunteers' blood (n = 10) with EO4 formulation

| Parameter | APTT [s] | |
|---|---|---|
| EO4 concentration (μg/ml) | control | EO4 |
| 7.5 | 34.5 ± 1.0 | 37.4 ± 2.4 |
| 15 | | 38.2 ± 1.6* |

Results are presented as means, and standard error. APTT- activated partial thromboplastin time.
*APTT (0) ≠ APTT (EO4), p=0.012
EO4 formulation in a concentration of 15 μg/ml exhibited in vitro anticoagulant properties for all determined parameters. Concentration of 7.5 μg/ml of EO4 formulation inhibited only CT.

Table 10. The rex-tem test results of the thromboelastometric study after incubation of the healthy volunteers' blood (n = 10) with EO4 formulation

| Parameter | CT [s] | | α [o] | | CFT [s] | |
|---|---|---|---|---|---|---|
| EO4 concentration (μg/ml) | control | EO4 | control | EO4 | control | EO4 |
| 7.5 | 63.1 ± 4.8 | 74.0 ± 5.3 | 69.4 ± 3.8 | 66.3 ± 1.7** | 124.3 ± 15.0 | 121.9 ± 9.8 |
| 15 | 63.1 ± 4.8 | 81.5 ± 6.7 | 69.4 ± 3.8 | 52.0 ± 3.7 | 124.3 ± 15.0 | 253.0 ± 34.5 |

Results are presented as means, and standard error. CT- coagulation time, $\alpha$ - inclination angle of thromboelastographic curve, CFT - clot formation time.

For the concentration of 7.5 μg/ml
CT (0) ≠ CT (EO4), $p<0.05$
For the concentration of 15 μg/ml
CT (0) ≠ CT (EO4), $p<0.03$
$\alpha$ (0) ≠ $\alpha$ (EO4), $p<0.02$
CFT (0) ≠ CFT (EO4), $p<0.01$

Presented effect of EO4 formulation on APTT parameter proves its anticoagulant properties. Effect of EO4 formulation on the analyzed parameters of thromboelastographic curve proves anticoagulant activity of the formulation going beyond the antiplatelet effect.

**Example 6**

Assessment of the impact of administration of polyphenolic formulation on the survival rate of rats with experimental diabetes.

Animals subjected to examination

In the experiment, male Sprague-Dawley rats weighing 250-300 g were used. Animals, both during breeding, and during experiment, were fed with soy-free feed, low in phytoestrogens.

Induction of diabetes, and division into experimental groups

Experimental diabetes was induced by intraperitoneal injection of streptozotocin at a dose of 70 mg / kg body weight. After injection, animals were labelled with numbered clips. 7 days after injection, the animals were weighed, and glucose in venous blood was measured. The condition to recognize an animal as suffering from diabetes, and to include it in the experiment was the simultaneous fulfilment of the following conditions:

- weight loss of at least 10%
- glycaemia value exceeding 200 mg%.

Animals meeting simultaneously the above criteria were randomly allocated to 4 experimental groups, 40 individuals being in each group. Experimental groups receiving the following doses of formulation were created:

- 0 mg of gallic acid / kg body weight / a day
- 2.5 mg of gallic acid / kg body weight / a day
- 5 mg of gallic acid / kg body weight / a day
- 10 mg of gallic acid / kg body weight / a day

In the study, standardization of the studied formulation on the gallic acid content was assumed, so the doses are expressed as the weight of total polyphenols in the formulation in terms of gallic acid. Later in this example, the studied polyphenolic formulation, obtained as specified in part 1 of example 1, is referred to as EO4.

Administration of EO4 formulation

Based on previous experience, it was demonstrated that the presence of the studied formulation, at the highest tested dose, in drinking water, does not affect the amount of liquid consumed by animals. On this basis, this way of administration of the studied formulation was adopted. Animals in the control group received pure drinking water.

Animals were placed in cages, no more than five individuals in one, (position of cages on the shelves in the animal living room was changed in a controlled manner). Every 7 days, the measurements of body weight were made. On this basis, the mass of the formulation, which animals in a given cage should consume in order to receive the specified dose, was calculated. Every day, the appropriate weight of the formulation was dissolved in 0.5 litres of drinking water and administered to the animals.

Observations were carried for 34 weeks. Every day, animal deaths were recorded, and every 7 days, their weight and blood glucose were recorded.

Results

In groups of animals supplemented with EO4, longer survival periods were observed, as illustrated by the data presented in Fig. 3, and Table 2

Fig. 3 shows the survival curves in animals supplemented with EO4 formulation after administration of four different doses of formulation: 0, 2.5, 5, and 10 mg / kg body weight per day, while Table 2 shows the average survival period of diabetic animals supplemented with EO4 formulation.

Table11 The average survival period of diabetic animals supplemented with EO4 formulation.

| Experimental group | Average survival period (95% confidence interval) [weeks] |
|---|---|
| 0 mg/kg b.w./d | 26.4 (23.4 - 29.5) |

(continued)

| Experimental group | Average survival period (95% confidence interval) [weeks] |
|---|---|
| 2.5 mg/kg b.w./d | 31.0 (29.2 - 32.8) |
| 5 mg/kg b.w./d | 31.3 (30.1 - 32.5) |
| 10mg/kg b.w./d | 28.5 (25.8 - 31.2) |

Comparison of survival curves with Gehan-Breslow-Wilcoxon test (corrected for multiple comparisons) shows significant differences for groups supplemented with EO4 at doses of 2.5, and 5 mg / kg bw / day compared to the placebo group.

Survival of the group supplemented with EO4 at the dose of 10 mg / kg bw / day did not differ significantly from the placebo group, which the data in Table 3 represent.

Table 12 Comparison of survival curves of diabetic animals supplemented with EO4 formulation.

| compared groups | p values - Gehan-Breslow-Wilcoxon test (after Bonferroni corrections for multiple comparisons) |
|---|---|
| 0 vs. 2.5 mg/kg b.w./d | $p<0.01$ |
| 0 vs. 5 mg/kg b.w./d | $p<0.01$ |
| 0 vs. 10 mg/kg b.w./d | n.s. |

Value summary of proportional hazard leads to similar conclusions (hazard ratio) (Table 4).

Table 4 Value summary of proportional hazard (hazard ratio) for survival curves of diabetic animals supplemented with EO4 formulation.

| compared groups | value of proportional hazard (95% confidence interval) |
|---|---|
| 0 vs. 2.5 mg/kg b.w./d | 3.10 (1.45 - 6.59) |
| 0 vs. 5 mg/kg b.w./d | 3.18 (1.50 - 6.74) |
| 0 vs. 10 mg/kg b.w./d | 1.89 (0.86 - 4.14) |

No effect of the formulation administration on body weight was observed, as illustrated by the data shown in Fig. 4 (change in body weight in animals supplemented with EO4). Administration of the drug had also no effect on the glycaemia of supplemented animals, as illustrated by the data presented in Fig. 5

Supplementation with the EO4 formulation at doses of 2.5, and 5 mg / kg b.w./ day significantly improves survival in diabetic animals without affecting the basic determined parameters describing the state, and condition of the body when diabetes is not treated. This suggests that EO4 formulation treats secondary effect of hyperglycaemia rather than glycaemic control *per se.*

**Example 7**

Determination of cytotoxicity

Determination of cytotoxicity for the formulation obtained according to the method described in Example 1 and referred herein as EO4, has been done on the basis of multiparameter automated HCS cytotoxicity test "Cell health assay," using the camera Thermo Scientific Cellomics ® ArrayScan making possible an automatic microscopic analysis, which allows to investigate the damage to cell structures caused by the analysed formulation. Thanks to the use of three fluorescent dyes (MitoTracker - Invitrogen, YO-PRO 1 - Invitrogen, Hoechst 33 342 - Sigma), assessment of the impact of the studied extracts on the size, and morphology of the nucleus, cytoplasmic membrane integrity, and mitochondrial activity was made. Tests were performed on 96-well plates using 5 cell lines (HepG2, HMEC-1, A549, Caco-2, 3T3). The measurements were taken using automatic pipetting station JANUS by Perkin Elmer. The formulation was examined at five concentrations, measurements were taken in duplicates in 5 independent series. Based on the raw experimental data, statistical analysis of significance of observed differences in average values for three selected measurement parameters, measuring the fluorescence intensity, and dyed surface in cells, using fluorescent dyes, was made. The

selected parameters illustrate:

1. Surface of the cell nucleus (Hoechst (Sigma)
2. Accumulation of YO-PRO1 dye (Invitrogen), indicating cytoplasmic membrane damage
3. Mitochondrial potential (MitoTracker - Invitrogen)

Then, the statistically significant differences for each tested concentration of each substance, which indicate morphological, and functional changes in cells, showing cytotoxic activity, were assigned with point values as shown in the diagram of assessment of the observed effects below.

Differences subject to the scoring:

1. Decrease, or increase in nucleus surface
2. Increase in the accumulation of YO-PRO1
3. Decrease in the mitochondrial potential

The following Table 13 illustrates the assigned point values given to the studied formulations in "Cell health assay" test.

Table 13. Point values adopted in "Cell health assay" test.

| Event | Points | Maximal number of points |
|---|---|---|
| Significant difference in one cell line | 0.2 for each event | 3 |
| The same difference in more than one cell line | 1 for each additional line | 12 |
| More than one difference in one line | 1 for each additional difference | 10 |

The following Table 14 illustrates the cytotoxic activity of polyphenolic formulation (EO4) based on the cumulative cytotoxicity index calculated for each of the studied substances on the basis of the results obtained in the multiparameter measurement "cell health assay".

Table 14. Measurement of cytotoxicity of the polyphenolic formulation with the use of naringin as a negative control, and kaempferol as a positive control.

| | 5 μg/ml | 10 μg/ml | 30 μg/ml | 40 μg/ml | 50 μg/ml | Total |
|---|---|---|---|---|---|---|
| Naringin | 0.0 | 0.0 | 0.2 | 1.4 | 0.2 | 1.8 |
| EO4 | 0.0 | 0.0 | 0.2 | 2.6 | 5.0 | 7.8 |
| Kaempferol | 2.6 | 1.4 | 8.4 | 8.4 | 6.2 | 27.0 |

The obtained results indicate that the studied polyphenolic formulation is not cytotoxic at concentrations lower than 30 μg / ml.

## Claims

1. Defatted extract from spent hops for use in antithrombotic treatment or antithrombotic prophylaxis in human or animal subjects, wherein from said extract lipophilic compounds, including prenylated flavonoids, have been removed.

2. Extract for use according to claim 1, wherein said treatment or said prophylaxis is associated with antiplatelet action.

3. Extract for use according to claim 1, wherein said treatment or said prophylaxis is associated with anticoagulant action.

4. Extract for use according to claim 1, wherein said treatment or said prophylaxis is associated with modulation of vascular endothelium activity.

5. Extract for use according to one of claims 1-4, wherein said treatment or said prophylaxis is against cardiovascular

disease.

6. Extract for use according to claim 5, wherein said disease is atherosclerosis, ischemic heart disease, aorta and peripheral vascular diseases, peripheral vein disease, venous thromboembolism.

7. Defatted extract from spent hops for use in cardiovascular protective treatment in the state of persistent hyperglycaemia in human or animal subjects, wherein from said extract lipophilic compounds, including prenylated flavonoids, have been removed.

## Patentansprüche

1. Entfetteter Auszug aus gegorenem Hopfen zum Einsatz in antithrombotischer Behandlung oder antithrombotischer Prophylaxe beim menschlichen oder tierischen Subjekt, in dem vom oben genannten Auszug die lipophilen Komponenten, prenylierte Flavonoide beinhaltend, entfernt wurden.

2. Auszug zum Einsatz nach Anspruch 1, in dem die genannte Behandlung oder genannte Prophylaxe mit thrombozytensegregationshemmender Wirkung verbunden ist.

3. Auszug zum Einsatz nach Anspruch 1, in dem die genannte Behandlung oder genannte Prophylaxe mit antikoagulativer Wirkung verbunden sind.

4. Auszug zum Einsatz nach Anspruch 1, in dem die genannte Behandlung oder genannte Prophylaxe mit der Modellierung der Aktivität des vaskulären Endothels verbunden sind.

5. Auszug zum Einsatz nach einem der Ansprüche 1-4, in dem die genannte Behandlung oder genannte Prophylaxe gegen Herz-Kreislauf-Erkrankungen gerichtet ist.

6. Auszug zum Einsatz nach Anspruch 5, in dem die genannte Krankheit Atherosklerose, ischämische Herzkrankheit, periphere arterielle Verschlusskrankheit, periphere Venenkrankheit, Venöser Thromboembolismus ist.

7. Entfetteter Auszug aus gegorenem Hopfen zum Einsatz in vorbeugender Herz-Kreislauf-Behandlung im Zustand einer permanenten Hyperglykämie beim menschlichen oder tierischen Subjekt, in dem vom oben genannten Auszug die lipophilen Komponenten, prenylierte Flavonoide beinhaltend, entfernt wurden.

## Revendications

1. Un extrait de houblon épuisé dégraissé pour utilisation dans un traitement antithrombotique ou une prophylaxie antithrombotique chez les sujets humains ou animaux, où dudit extrait des composés lipophiles, y compris les flavonoïdes prénylés, ont été enlevés.

2. L'extrait pour utilisation selon la revendication 1, où ledit traitement ou ladite prophylaxie est associée à l'action antiplaquettaire.

3. L'extrait pour utilisation selon la revendication 1, où ledit traitement ou ladite prophylaxie est associée à l'action anticoagulante.

4. L'extrait pour utilisation selon la revendication 1, où ledit traitement ou ladite prophylaxie est associée à la modulation de l'activité de l'endothélium vasculaire.

5. L'extrait pour utilisation selon l'une quelconque des revendications 1-4, où ledit traitement ou ladite prophylaxie est contre les maladies cardiovasculaires.

6. L'extrait pour utilisation selon la revendication 5, où ladite maladie est l'athérosclérose, la maladie cardiaque ischémique, la maladie aortique et les maladies vasculaires périphériques, la maladie veineuse périphérique, la thromboembolie veineuse.

7. L'extrait de houblon épuisé dégraissé pour utilisation dans un traitement de protection cardiovasculaire dans l'état de l'hyperglycémie persistante chez les sujets humains ou animaux, où dudit extrait des composés lipophiles, y compris les flavonoïdes prénylés, ont été enlevés.

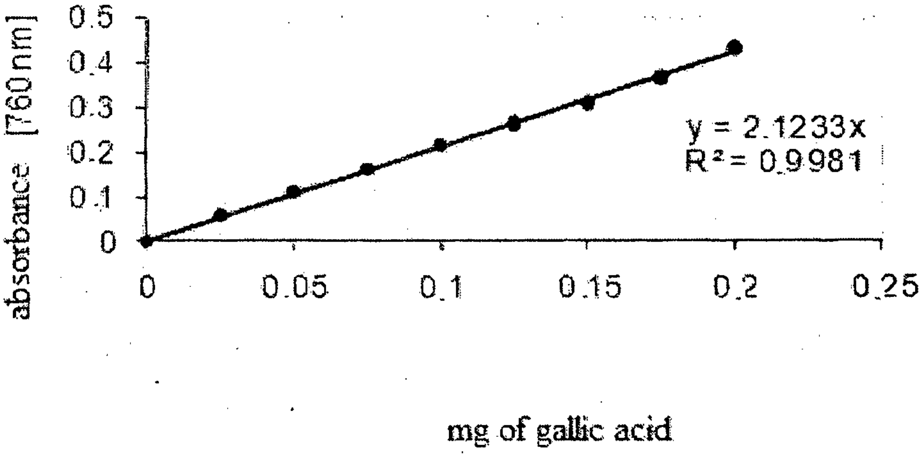
0.5
0.4
0.3
0.2
0.1
0
absorbance [760 nm]
y = 2.1233x
R² = 0.9981
0
0.05
0.1
0.15
0.2
0.25
mg of gallic acid

Fig. 1

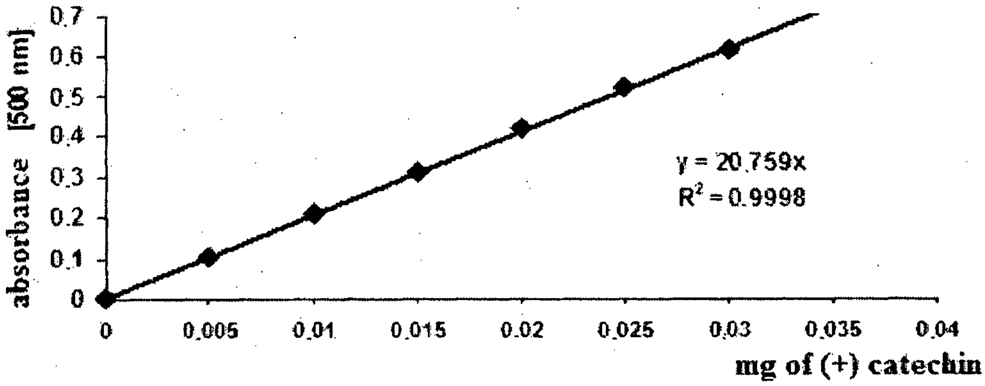
0.7
0.6
0.5
0.4
0.3
0.2
0.1
0
absorbance [500 nm]
y = 20.759x
R² = 0.9998
0
0.005
0.01
0.015
0.02
0.025
0.03
0.035
0.04
mg of (+) catechin

Fig. 2

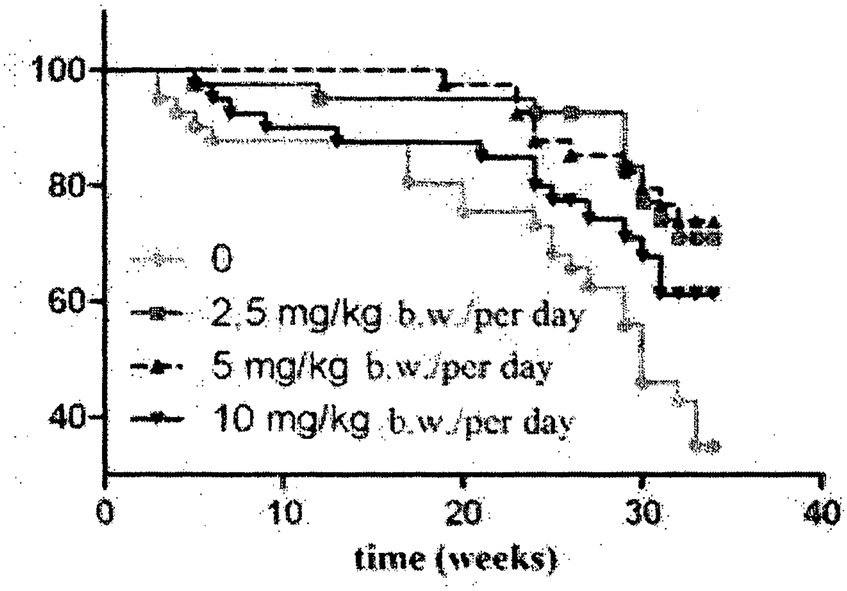


Y axis: Cumulative survival rate

Fig. 3

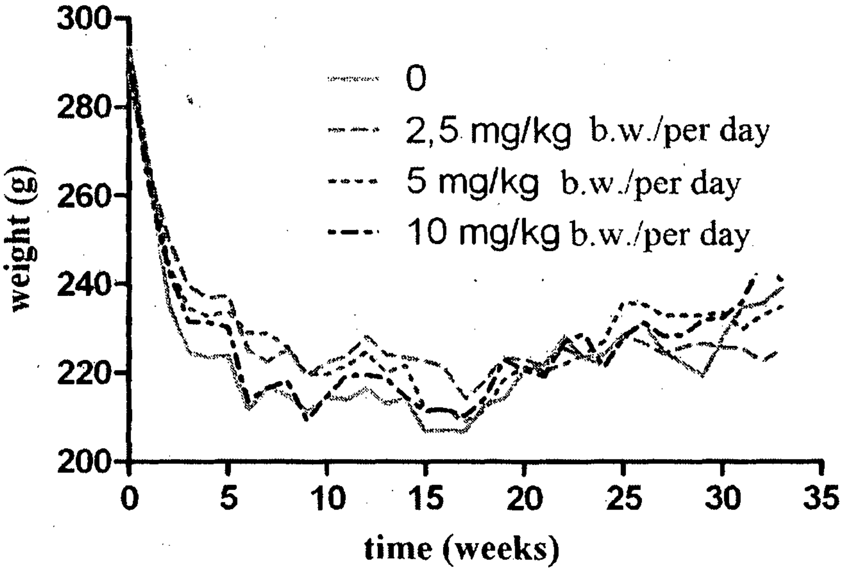


Fig. 4

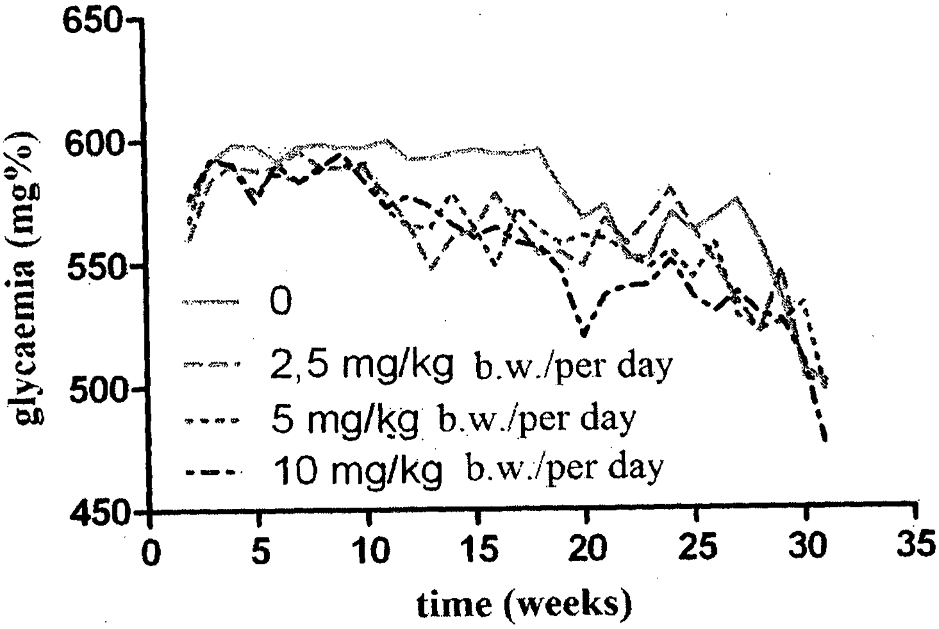
650
600
550
500
450
glycaemia (mg%)
0
5
10
15
20
25
30
35
time (weeks)
0
2,5 mg/kg b.w./per day
5 mg/kg b.w./per day
10 mg/kg b.w./per day

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1543834 A
- US 20050042318 A
- WO 8300701 A1
- EP 19939350 A1
- WO 2009023710 A
- US 20090291071 A